# EUROPÄISCHE PATENTSCHRIFT

(11) **EP 1 682 023 B1**
(45) Veröffentlichungstag und Bekanntmachung des Hinweises auf die Patenterteilung: **09.12.2009**
(21) Anmeldenummer: 04790982.5
(22) Anmeldetag: 28.10.2004
(51) Int. Cl.: A61B 18/00

(54) **VORRICHTUNG ZUR KOAGULATION VON GEWEBE**
APPARATUS FOR COAGULATING TISSUE
DISPOSITIF DE COAGULATION DE TISSU

(30) Priorität: 30.10.2003 DE 10350709
(43) Veröffentlichungstag der Anmeldung: 26.07.2006
(73) Patentinhaber: Erbe Elektromedizin GmbH, 72072 Tübingen (DE)
(72) Erfinder: SCHNITZLER, Uwe, 72074 Tübingen (DE); SCHÄLLER, Daniel, 72074 Tübingen (DE)
(74) Vertreter: Bohnenberger, Johannes
(86) Internationale Anmeldenummer: PCT/EP2004/012212
(87) Internationale Veröffentlichungsnummer: WO 2005/041800

(56) Entgegenhaltungen:
- US-A- 2 828 748
- US-A- 5 244 462
- US-A- 6 039 736
- US-A- 6 142 995
- US-B1- 6 197 026

## Beschreibung

Die Erfindung betrifft eine Vorrichtung zur Koagulation von Gewebe, umfassend eine Elektrode, die mit einem HF-Generator zur Erzeugung eines hochfrequenten Stromes verbunden ist und ein Rohr, eine schlauchförmige Sonde oder dergleichen Gaszuführungseinrichtung zum Zuführen von Argon oder dergleichen inertem Gas aus einer Mündung der Gaszuführungseinrichtung in einen Raum zwischen der Elektrode und dem Gewebe mit einer vorbestimmten Strömungsrichtung.

Eine derartige Vorrichtung ist beispielsweise aus der DE 41 390 29 A1 bekannt. Bei dieser Vorrichtung strömt das Gas axial zur Elektrode aus der Mündung der Gaszuführungseinrichtung aus und die Elektrode ist in der Mündung zurückversetzt angeordnet, so dass das Plasma bevorzugt in einer axialen Richtung zur Gaszuführungseinrichtung entsteht. Besonders dann, wenn bei einer endoskopisch in einer Körperhöhle durchgeführten Operation beengte Verhältnisse vorliegen, ist es schwierig, solche Gewebestellen zu koagulieren, die seitlich, also in radialer Richtung zur Mündung liegen.

Aus der DE 198 202 40 C2 ist eine Vorrichtung zur Koagulation von Gewebe bekannt, bei welcher die Elektrode vollständig innerhalb einer schlauchförmigen Sonde liegt, wobei die schlauchförmige Sonde einen rings um ihren Umfang schraubenförmig verlaufenden Schlitz derart aufweist, dass das zugeführte Gas und ebenso das Plasma in radialer Richtung zur Sonde austreten. Problematisch ist hierbei zum einen die Herstellung derartiger Sonden aufgrund der beengten Raumverhältnisse. Zum anderen kann relativ leicht eine Beschädigung des Sondenmaterials durch die hohen Temperaturen des Plasmas auftreten.

US 5,244,462 offenbart eine Vorrichtung nach dem Oberbegriff des Anspruchs 1.

Der Erfindung liegt die Aufgabe zugrunde, eine Vorrichtung der eingangs genannten Art dahingehend weiterzubilden, dass in einfacher und bei Benutzung sicherer Weise eine Richtung des Plasmastrahls abweichend von einer axialen Richtung vorgebbar ist.

Diese Aufgabe wird bei einer Vorrichtung der eingangs genannten Art dadurch gelöst, dass am distalen Ende eine aus elektrisch isolie dem Material ausge-bildete Leiteinrichtung zum Richten und Leiten des Gases und/oder des Plasmas derart angeordnet ist, das die Elektrode die leiteinrichtung trägt und wenigstens ein Teil des strömenden Gases/Plasmas in die vorbestimmte Richtung umgeleitet wird.

Ein wesentlicher Punkt der Erfindung liegt darin, dass die Elektrode selbst in den mechanischen Gesamtaufbau der Vorrichtung dadurch konstruktiv einbezogen wird, dass sie gewissermaßen einen Teil der Gaszuführungseinrichtung, nämlich die Leiteinrichtung trägt. Die Vorzugsrichtung des Gases bzw. des Plasmas wird somit durch die Leiteinrichtung bestimmt. An dieser Stelle sei angemerkt, dass innerhalb eines vollständig mit einem Inertgas gefülltem Raumes die Richtung, in welcher sich das Plasma ausbildet, nicht durch die Gasströmung beeinflusst wird. Dadurch aber, dass das Plasma immer in der Strecke auftritt, welche den geringsten Gesamtwiderstand aufweist und eine vollständig homogene Füllung des Raumes selbst in einer Körperhöhle kaum erreichbar ist, kann über die Leiteinrichtung einerseits der Gasstrom und damit die Gaskonzentration innerhalb des Raumes bestimmt und andererseits über eine Verlängerung des durch das Plasma zu überbrückenden Weges von der Elektrode zum Gewebe die angestrebte Richtungsumleitung erfolgen.

Erfindugsgemäß besteht die Leiteinrichtung aus einem elektrisch isolierenden Material, wodurch die soeben erwähnte Wegveränderung erleichtert wird.

Weiterhin bestehen die Leiteinrichtungen vorzugsweise aus einem temperaturbeständigem Material derart, dass während einer Operation auch bei längerer Beaufschlagung der Leiteinrichtung mit dem Plasma keine schädigenden Veränderungen an dem Material auftreten. Als Material eignet sich insbesondere eine Keramik, die beispielsweise aufgespritzt oder durch Tauchen aufgebracht sein kein.

Die Elektrode ist vorzugsweise stab- oder drahtförmig in an sich bekannter Weise ausgebildet, wobei die Leiteinrichtung vorzugsweise axialsymmetrisch rings um die Elektrode derart angeordnet ist, dass das Gas/Plasma im wesentlichen radial zur Mündung der Gaszuführungseinrichtung in den Raum strömt. Auf diese Weise wird erreicht, dass die Vorrichtung bei einer endoskopischen Operation innerhalb einer Körperhöhle nicht gedreht werden muss, um radial zur Mündung liegende Gewebebereiche zu koagulieren. Es ist lediglich notwendig, mit der Vorrichtung in die Nähe der zu koagulierenden Gewebestellen zu kommen, da sich das Plasma (wie oben erläutert) den kürzesten und damit widerstandsärmsten Weg sucht. Der Plasmastrom wandert erst dann unter Verlängerung der Plasmastrecke, wenn das behandelte Gewebe austrocknet und somit wiederum einen höheren Widerstand bekommt.

Die Leiteinrichtung ist vorzugsweise auf ihrer der Mündung zugewandeten Seite konkav ausgebildet, wodurch sich in besonders einfacher Weise eine strömungsgünstige Umlenkung des Gasstromes erzielen lässt.

Zur Verhinderung mechanischer Verletzungen bei Berührung des Gewebes ist die Leiteinrichtung auf ihrer, der Mündung abgewandten Seite abgerundet. Die Leiteinrichtung bildet somit gleichzeitig eine Schutzvorrichtung gegen eine direkte Berührung zwischen Elektrode und Gewebe, die bekanntlich fatale Folgen haben könnte.

Die Elektrode ist bei einer bevorzugten Ausführungsform der Erfindung relativ zur Mündung derart verschiebbar ausgebildet, dass die Leiteinrichtung in einem zurückgezogenen Zustand die Mündung im wesentlichen dicht verschließt. Dadurch kann gewährleistet werden, dass beim Einführen der Sonde keine Körperflüssigkeit oder andere Verschmutzungen in die Gaszuführungseinrichtung eintreten können.

Nachfolgend werden bevorzugte Ausführungsformen der Erfindung anhand von Abbildungen näher beschrieben. Hierbei zeigen
- Fig. 1: eine perspektivische Ansicht einer ersten bevorzugten. Ausführungsform der Erfindung mit schematisiert angedeuteten Peripheriegeräten,
- Fig. 2: eine zweite bevorzugte Ausführungsform der Erfindung in einer Darstellung ähnlich der nach Fig. 1 und
- Fig. 3: eine dritte Ausführungsform der Erfindung in einer Darstellung entsprechend der nach Fig. 2.

In der nachfolgenden Beschreibung werden für gleiche und gleichwirkende Teile dieselben Bezugsziffern verwendet.

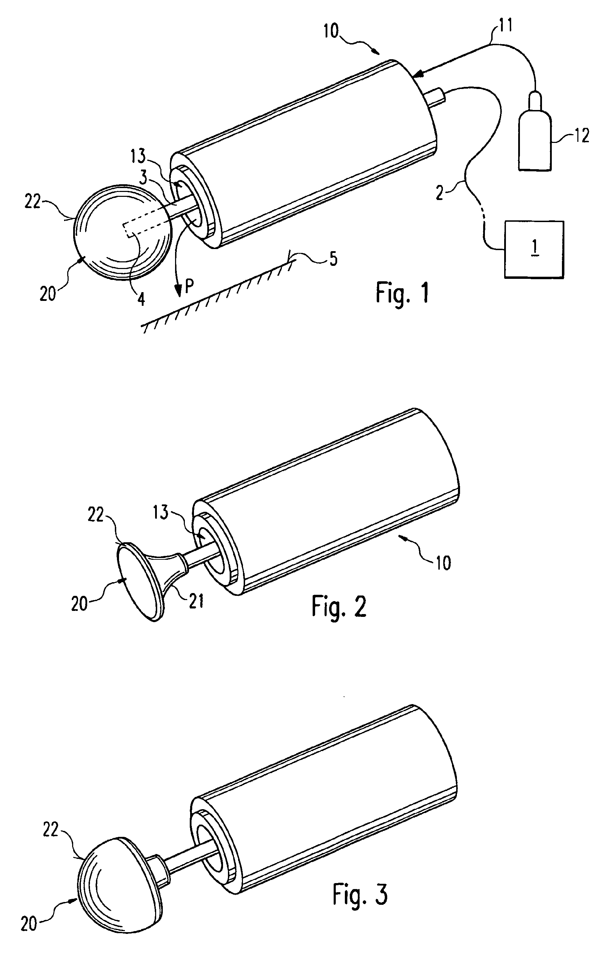

In Fig. 1 ist ein Endstück einer Sonde aufgezeigt, die eine Gaszuführungseinrichtung 10 umfasst, die rohrförmig ausgebildet ist und deren Lumen über eine Leitung 11 an eine Gasquelle 12 angeschlossen ist. In der Gaszuführungseinrichtung 10 ist eine Elektrode 3 (diese wird üblicherweise aus Wolfram gefertigt) im wesentlichen koaxial angeordnet, die über eine Stromleitung 2 mit einem HF-Generator verbunden ist. Ein distales Ende 4 der Elektrode 3 ragt aus einer Mündung 13 der Gaszuführungseinrichtung hervor.

Auf dem distalen Ende 4 der Elektrode 3 ist bei der in Fig. 1 gezeigten Ausführungsform der Erfindung ein kugelförmiges Keramikteil angebracht, das eine Leiteinrichtung 20 bildet. Edelgas aus der Gasquelle 12, welches aus der Mündung 13 austritt, wird durch diese Anordnung entlang der Richtung des Pfeiles P abgelenkt. Wenn weiterhin die Anordnung in der Nähe und parallel zu einer Gewebeoberfläche 5 liegt, so wird durch die Leiteinrichtung 20 in Zusammenwirkung mit dem Ende der Gaszuführungseinrichtung 10 im Bereich deren Mündung 13 ein verengter Raum derart gebildet, dass bei Ionisierung des zugeführten Edelgases durch einen aus dem Generator 1 kommenden Hochfrequenzstrom das entstehende Plasma zwischen der Elektrode 3 und der Gewebeoberfläche 5 als kürzesten Weg eine radial zur Elektrode 3 verlaufende Bahn vorfindet. Dadurch wird die Leiteinrichtung 20 nicht nur zur Bestimmung der Strömungsrichtung des zugeführten Edelgases sondern auch zum "Leiten" des Plasmas.

Die in Fig. 2 gezeigte Ausführungsform der Erfindung unterscheidet sich von der nach Fig. 1 dadurch, dass die Leiteinrichtung 20 nicht kugelförmig, sondern in Form eines Ventils für eine Brennkraftmaschine geformt ist und einen konkav ausgebildeten inneren Abschnitt 21 in dem Bereich aufweist, welcher der Mündung 13 der Gaszuführungseinrichtung 10 gegenüberliegt. Das zur Gaszuführungseinrichtung 10 distale Ende der Leiteinrichtung ist abgeflacht. Der Übergangsbereich zwischen dem abgeflachten distalen Abschnitt und dem inneren Abschnitt 21 weist eine Abrundung 22 derart auf, dass bei Berührung der Gewebeoberfläche 5 keine mechanische Verletzung des Gewebes auftreten kann.

Die in Fig. 3 gezeigte Ausführungsform der Erfindung unterscheidet sich von der nach Fig. 2 dadurch, dass anstelle eines flachen distalen Abschnittes der Leitvorrichtung 20 dieser Abschnitt kugelförmig ist, und somit insgesamt eine Abrundung 22 bildet, was wiederum die Verletzungsgefahr vermindert.

Die Elektrode 3 kann zurückziehbar bzw. die Mündung 13 vorschiebbar derart ausgebildet sein, dass im zurückgezogenen Zustand der Elektrode 3 die Leiteinrichtung 20 auf der Mündung 13 sitzt. In diesem Zustand wird der Gefahr vorgebeugt, dass bei einem Einschieben der Gaszuführungseinrichtung 10 bzw. einer entsprechend ausgestalteten Sonde Körperflüssigkeit oder dergleichen in das Lumen der Gaszuführungseinrichtung 10 eintritt, da die Mündung 13 ja verschlossen ist.

### Bezugszeichenliste

- 1: HF-Generator
- 2: Stromleitung
- 3: Elektrode
- 4: Distales Ende
- 5: Gewebeoberfläche
- 10: Gaszuführungseinrichtung
- 11: Leitung
- 12: Gasquelle
- 13: Mündung
- 20: Leiteinrichtung
- 21: Innerer Abschnitt
- 22: Abrundung

## Patentansprüche

1. Vorrichtung zur Koagulation von Gewebe, umfassend
eine Elektrode (3) die mit einem HF-Generator (1) zur Erzeugung eines hochfrequenten Stroms verbunden ist und
ein Rohr, eine schlauchförmige Sonde oder dergleichen Gaszuführungseinrichtung (10) zum Zuführen von Argon oder dergleichen inertem Gas aus einer Mündung (13) der Gaszuführungseinrichtung (10) in einen Raum zwischen der Elektrode (3) und dem Gewebe (5) derart, dass zwischen der Elektrode (3) und dem Gewebe (5) ein Plasma entsteht,
wobei die Elektrode (3) mit einem distalen Ende (4) aus der Gaszuführungseinrichtung (10) hervorsteht,
**dadurch gekennzeichnet, dass**
am distalen Ende (4) der Elektrode (3) eine aus elektrisch isolierendem Material ausgebildete Leiteinrichtung (20) zum Richten und Leiten des Gases und/oder Plasmas derart angeordnet ist,
dass die Elektrode (3) die Leiteinrichtung trägt
und dass wenigstens ein Teil des strömenden Gases und/oder des Plasmas in eine vorbestimmte Richtung umgeleitet wird.

2. Vorrichtung nach einem der vorhergehenden Ansprüche,
**dadurch gekennzeichnet, dass** die Leiteinrichtung aus einem temperaturbeständigen Material besteht.

3. Vorrichtung nach eine der Ansprüche 1 oder 2,
**dadurch gekennzeichnet, dass** das Material Keramik ist.

4. Vorrichtung nach einem der vorhergehenden Ansprüche,
**dadurch gekennzeichnet, dass** die Elektrode (3) stab- oder drahtförmig ausgebildet und die Leiteinrichtung (20) axialsymmetrisch rings um die Elektrode (3) derart ausgebildet ist, dass das Gas im Wesentlichen radial zur Mündung (13) der Gaszuführungseinrichtung (10) in den Raum strömt.

5. Vorrichtung nach einem der vorhergehenden Ansprüche,
**dadurch gekennzeichnet, dass** die Leiteinrichtung (20) auf ihrer der Mündung (13) zugewandten Seite (21) konkav ausgebildet ist.

6. Vorrichtung nach einem der vorhergehenden Ansprüche,
**dadurch gekennzeichnet, dass** die Leiteinrichtung (20) zur Verhinderung mechanischer Verletzungen bei Berührung des Gewebes (5) abgerundet ist.

7. Vorrichtung nach einem der vorhergehenden Ansprüche,
**dadurch gekennzeichnet, dass** die Elektrode (3) relativ zur Mündung (13) derart verschiebbar ausgebildet ist, dass die Leiteinrichtung (20) in einem zurückgezogenen Zustand die Mündung (13) im wesentlichen dicht verschließt.

## Claims

1. Apparatus for coagulating tissue comprising
an electrode (3) connected to an HF generator (1) for generating a high-frequency current and
a tube, a tubular probe or similar gas-delivering device (10) for delivering argon or a similar inert gas from an outlet (13) of said gas-delivering device (10) in a space between the electrode (3) and the tissue (5) such that a plasma forms between the electrode (3) and the tissue (5),
wherein a distal end (4) of said electrode (3) projects out of said gas-delivering device (10),
**characterised in that**
a guiding device (20) made of an electrically insulating material for directing and guiding said gas and/or plasma is disposed at the distal end (4) of the electrode (3) such that the electrode (3) bears the guiding device
and that least a part of the stream of gas and/or the plasma is diverted into a predetermined direction.

2. Apparatus according to any one of the preceding claims, **characterised in that** the guiding device comprises a thermally stable material.

3. Apparatus according to either claim 1 or claim 2, **characterised in that in that** the material is ceramic.

4. Apparatus according to any one of the preceding claims, **characterised in that** the electrode (3) is rod-shaped or wire-shaped and the guiding device (20) is disposed in an axially symmetrical way around the electrode (3) such that the gas flows into the space substantially radially with respect to the outlet (13) of the gas-delivering device (10).

5. Apparatus according to any one of the preceding claims, **characterised in that** the guiding device (20) is concave on its side (21) facing the outlet (13) facing the outlet (13).

6. Apparatus according to any one of the preceding claims, **characterised in that** the guiding device (20) is rounded to prevent mechanical damage on contact with the tissue (5).

7. Apparatus according to any one of the preceding claims, **characterised in that** the electrode (3) is displaceable relative to the outlet (13) such that, when in retracted state, the guiding device (20) closes the outlet (13) in a substantially leakproof manner.

## Revendications

1. Dispositif pour la coagulation d'un tissu, comprenant :
une électrode (3) qui est connectée à un générateur HF (1) dans le but de générer un courant à hautes fréquences, et
un tuyau, une sonde tubulaire ou un dispositif d'alimentation en gaz similaire (10) pour acheminer de l'argon ou un gaz inerte similaire depuis une embouchure (13) du dispositif d'alimentation en gaz (10) dans un espace compris entre l'électrode (3) et le tissu (5) de manière à obtenir un plasma entre l'électrode (3) et le tissu (5),
l'électrode (3) dépassant du dispositif d'alimentation en gaz (10) par le biais d'une extrémité distale (4),
**caractérisé en ce que**
à l'extrémité distale (4) de l'électrode (3), on aménage un dispositif de guidage (20) formé d'un matériau électriquement isolant pour diriger et guider le gaz et/ou le plasma de sorte que l'électrode (3) porte le dispositif de guidage
et qu'au moins une partie du gaz qui s'écoule et/ou du plasma soit dévié dans une direction prédéterminée.

2. Dispositif selon l'une quelconque des revendications précédentes, **caractérisé en ce que** le dispositif de guidage est constitué d'un matériau résistant à la température.

3. Dispositif selon l'une quelconque des revendications 1 ou 2,
**caractérisé en ce que** le matériau est une céramique.

4. Dispositif selon l'une quelconque des revendications précédentes,
**caractérisé en ce que** l'électrode (3) est élaborée sous la forme d'une tige ou d'un fil et le dispositif de guidage (20) est réalisé selon une symétrie axiale autour de l'électrode (3) de sorte que le gaz s'écoule dans l'espace de manière sensiblement radiale par rapport à l'embouchure (13) du dispositif d'alimentation en gaz (10).

5. Dispositif selon l'une quelconque des revendications précédentes,
**caractérisé en ce que** le dispositif de guidage (20) se présente sous une forme concave sur sa face (21) tournée vers l'embouchure (13).

6. Dispositif selon l'une quelconque des revendications précédentes,
**caractérisé en ce que** le dispositif de guidage (20) est arrondi pour empêcher des lésions mécaniques en cas de contact avec le tissu (5).

7. Dispositif selon l'une quelconque des revendications précédentes,
**caractérisé en ce que** l'électrode (3) est réalisée de manière à coulisser par rapport à l'embouchure (13) de sorte que le dispositif de guidage (20) ferme de manière sensiblement étanche l'embouchure (13) à l'état retiré.
